# EUROPEAN PATENT APPLICATION

(11) **EP 3 418 287 A1**
(43) Date of publication of application: **26.12.2018**
(21) Application number: 17177168.6
(22) Date of filing: 21.06.2017
(51) Int. Cl.: C07H 3/06, C08B 37/00

(54) **CHEMICAL RELEASE OF ASPARAGINELINKED GLYCANS**

(71) Applicant: Universität für Bodenkultur Wien, 1180 Wien (AT)
(72) Inventor: ALTMANN, Friedrich, 1140 Vienna (AT); FIGL, Rudolf, 1120 Vienna (AT)
(74) Representative: Gassner, Birgitta

(57) **Abstract**

The present invention relates to a method for producing reduced glycans from a glycopeptide or glycoprotein comprising reacting the glycopeptide or glycoprotein with borohydride in the presence of a base at a temperature of at least 50 °C for a period sufficient to obtain the reduced glycans.

## Description

### Field of the Invention

The present invention relates to the field of chemically releasing glycans from glycoproteins or Glycopeptides.

### Background Art

In 1968, D.M. Carlson introduced a protocol for reductive β-elimination of O-glycans that is still in use nowadays (Carlson, D.M. 1968). This 50 mM NaOH, 1 M NaBH₄ at about 50 °C for 16 h protocol was later also applied to N-glycoproteins (Rasilo, M.L. and Renkonen, O. 1981), even though in a study on fetuin O-glycans no concomitant release of N-glycans was noticed (Spiro, R.G. and Bhoyroo, V.D. 1974). However, these mild conditions were found to liberate 60 % of N-glycans (Ogata, S. and Lloyd, K.O. 1982). Later, it was argued that much smaller percentages were actually released as oligosaccharides and that small glycopeptides may have led to overestimation of the success (Debray, H., Strecker, G., et al. 1984). No more than 20 % of fetuin N-glycans was liberated under these conditions but apparently with the sialic acids remaining on the glycans (Hounsell, E.F., Pickering, N.J., et al. 1984). This was confirmed later in a study that obtained 95 % release of ribonuclease B N-glycans using stronger alkali and higher temperature (Argade, S.P., Daves, G.D., Jr., et al. 1989). Later on, unintended low yield "bycatch" of N-glycans by the Carlson conditions was repeatedly observed (Karlsson, N.G. and Packer, N.H. 2002, Schulz, B.L., Packer, N.H., et al. 2002, Yu, G., Zhang, Y., et al. 2010). A chemical method for selectively releasing O- and N-glycans was developed but has been largely outperformed by use of the peptide N-glycosidase F (Likhosherstov, L.M., Novikova, O.S., et al. 1990).

Release of O-glycans by reductive β-elimination has become routine in many glyco-analytical laboratories and concomitant release of N-glycans has repeatedly been observed. However, e.g. for the production of reference glycans, a method for the production of sizeable amounts of N-glycans, cheaper than enzymatic release, as well as of reduced glycans would be desirable.

### Summary of invention

Thus it is an object of the invention to provide a cheap and selective method for obtaining N-glycans and reduced glycans.

The object is solved by the subject matter of the present invention.

The present invention relates to a method for producing reduced glycans from a glycopeptide or glycoprotein comprising reacting the glycopeptide or glycoprotein with borohydride in the presence of a base at a temperature of at least 50 °C for a period sufficient to obtain the reduced glycans.

In one embodiment of the invention the reduced glycans are of general formula I and/or II 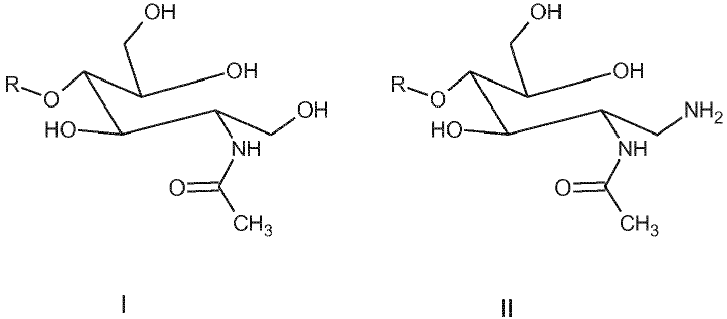 wherein R denotes a glycan residue as defined herein.

In some embodiments of the invention the OH-groups at C6 and/or C3 of compounds I or II are substituted by fucose.

One embodiment of the invention relates to the method as described herein, wherein the temperature is about 55 to 90°C, or about 60 to 80 °C. Specifically, the temperature may be 55 °C, 60 °C, 65 °C, 70 °C, 75 °C, 80 °C, 85 °C, or 90 °C.

One embodiment of the invention relates to the method as described herein, wherein the borohydride is used in a concentration of about 0.5 to 3.0 M, or of about 0.8 to 2.0 M. Specifically, the borohydride is used in a concentration of about 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 M. Preferably, the borohydride is used in a concentration of about 1.0 M or 2.0 M.

One embodiment of the invention relates to the method as described herein, wherein the base is used in a concentration of about 0.01 to 3.0 M, or of about 0.05 to 2.0 M. Specifically, the base is used in a concentration of about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 M. Preferably, the base is used in a concentration of about 0.5 M.

A further embodiment of the invention relates to the method as described herein, wherein the base is a strong base, e.g., sodium hydroxide (NaOH), lithium hydroxide (LiOH), potassium hydroxide (KOH), rubidium hydroxide (RbOH), cesium hydroxide (CsOH), magnesium hydroxide (Mg(OH)₂, calcium hydroxide (Ca(OH)₂), strontium hydroxide (Sr(OH)₂), or barium hydroxide (Ba(OH). Preferably, the base is NaOH, KOH, or LiOH.

A further embodiment of the invention relates to the method as described herein, wherein deoxygenated compound 2-amino-3-(glyco-)carbamoyl-propanol or 2,(glyco-)4-diamino-butyric acid are is obtained.

One embodiment of the invention relates to a compounds of general formula II III, or IV 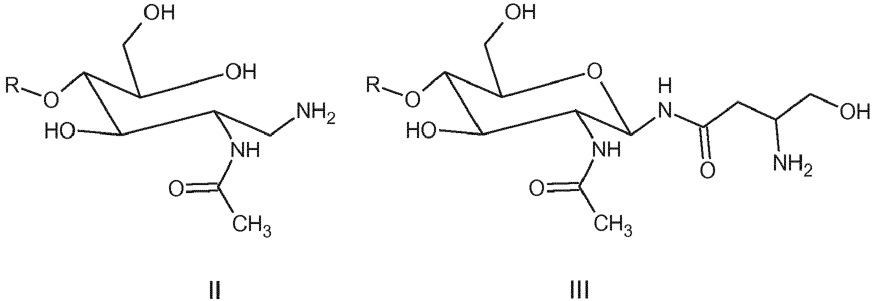 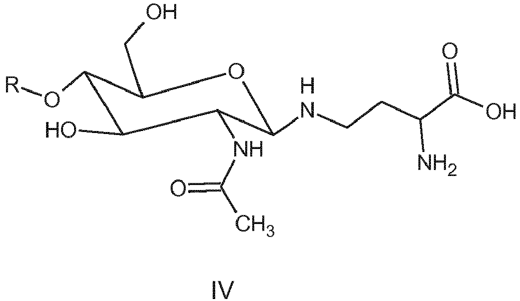 wherein R is a glycan residue.

In some embodiments of the invention the OH-groups at C6 and/or C3 of compounds II, III, or IV, are substituted by fucose.

One embodiment of the invention relates to a glycan array comprising:
a substrate, and at least four glycans, wherein at least one glycan is a compound of general formula I, II or III. Preferably, the compound is of general formula II or III.

A further embodiment of the invention relates to a glycan array as described herein, wherein each glycan is attached to said substrate by a linker.

A further embodiment of the invention relates to the glycan array as described herein, wherein said linker is selected from the group consisting of -O(CH₂)₂CH₂NH₂ and -O(CH₂)₃NHCOCH₂(OCH₂CH₂)₆NH₂.

One embodiment of the invention relates to the use of a glycan array as described herein in biochemical research or in biomedical applications.

A further embodiment of the invention relates to the use as described herein, wherein the binding of proteins to glycans of different structure is characterized.

A further embodiment of the invention relates to a stationary phase for affinity chromatography, wherein a compound of general formula II or III or IV is bound to the chromatography matrix.

### Brief description of drawings

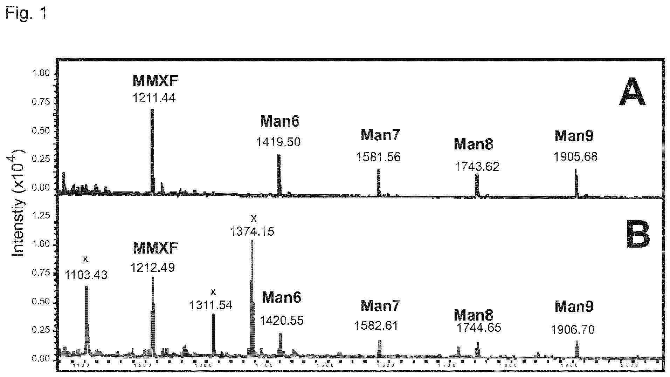

Fig. 1: MALDI-TOF MS of N-glycans from white beans released A) by PNGase A and B) by reductive alkaline hydrolysis. Peaks marked with an x are regarded as impurities. The 1311 Da peak was later identified as glyco-asparaginol (= Asn with a reduced carboxy group).

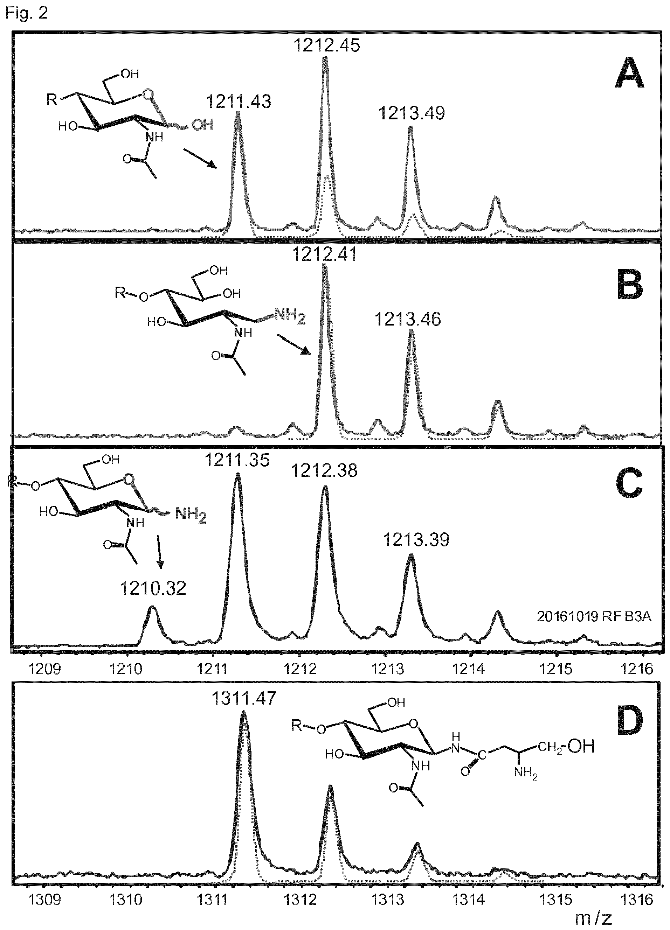

Fig. 2: MALDI-TOF MS peak profiles of the glycan MMXF (Man₃XylFucGlcNAc₂) generated by alkaline hydrolysis under classical Carlson conditions (A), with 2 M borohydride (B), classical Carlson conditions but workup in alkaline buffer (C). Panel D shows the 1311 Da peak, which was identified as N-glycan with a carboxyl-reduced asparagine (2-amino-3-(glyco-)carbamoyl-propanol). Dotted lines simulate the theoretical isotope pattern tailored to the first peak. For panel A this also represents the pattern observed after enzymatic release with PNGase A.

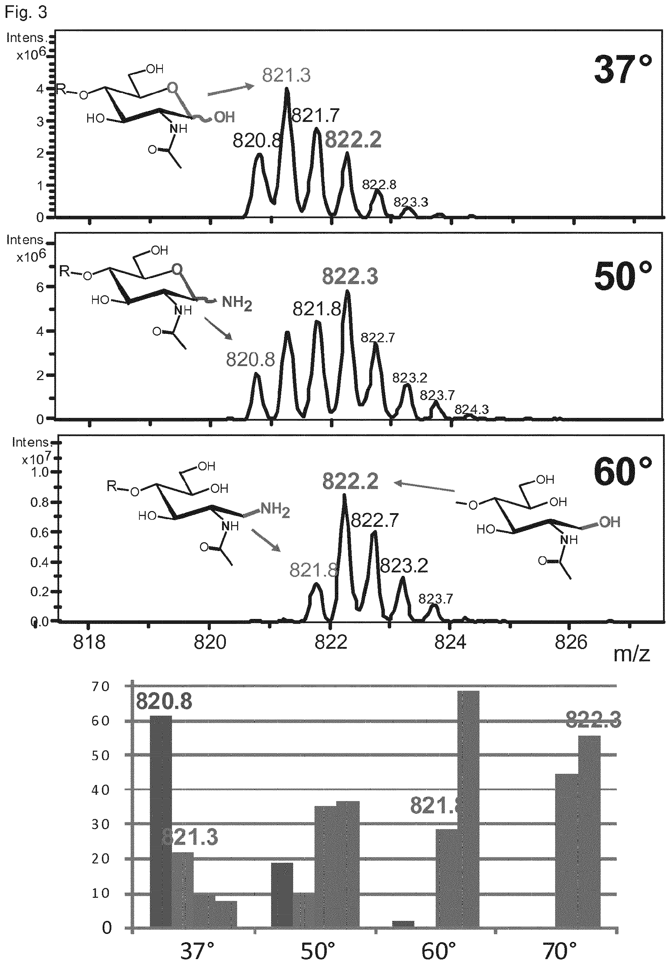

Fig. 3: Isotope patterns of the fibrin glycan A⁴A⁴ after chemical release at different temperatures under classical Carlson conditions. The graphics at the bottom shows the calculated percentages of the four major products glycosylamine (exact mass m/z = 820.8), reducing N-glycan (821.3), 1-amino-glycitol (821.8) and the expected reduced form glycit (822.3).

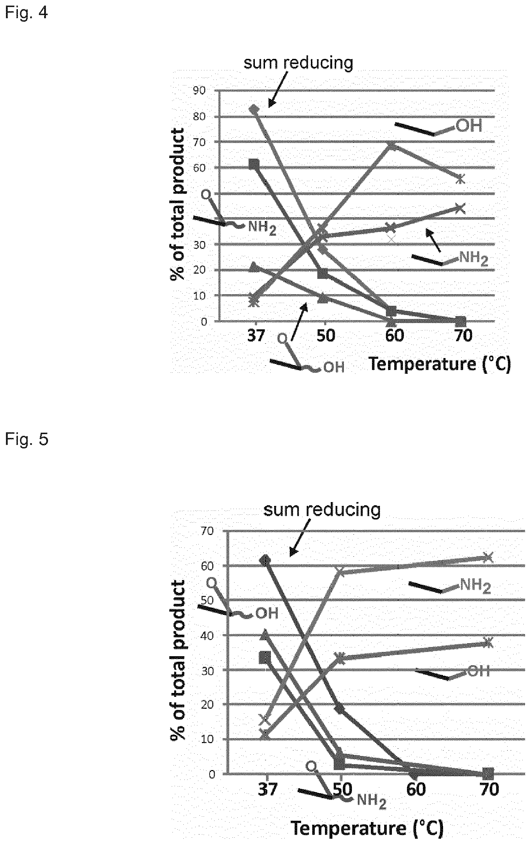

Fig. 4: Influence on temperature on the distribution of products. The percentage of each product obtained by reductive alkaline hydrolysis of fibrin glycopeptides after 16 h incubation in 50 mM NaOH, 1 M NaBH₄ at different temperatures. While the reducing N-glycan (in red) is just a minor component, it becomes a major product upon acidification and decay of the glycosylamine (purple).
Fig. 5: Relative yield of fibrin N-glycan derivatives after 16 h incubation in 50 mM NaOH, 2 M NaBH₄ at different temperatures. Note the predominance of 1-amino-glycitol (blue).

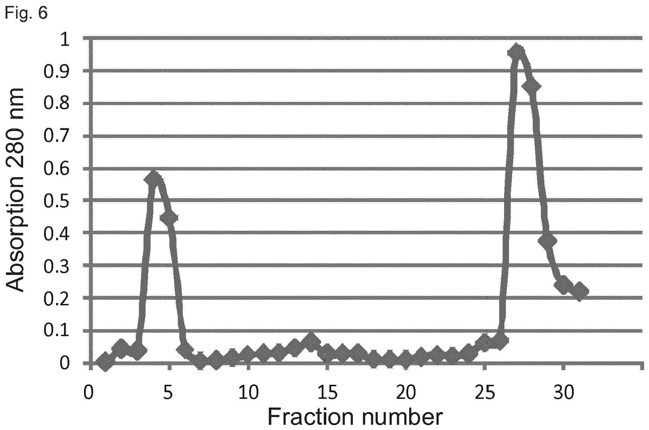

Fig. 6: Fractionation of chemically released glycans by cation exchange chromatography. High salt elution buffer was applied at about fraction 23.

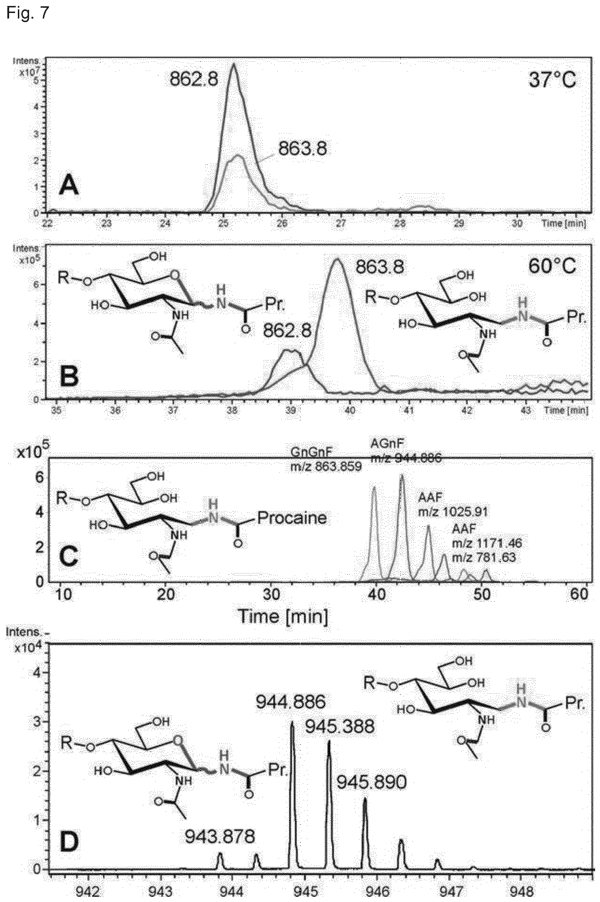

Fig. 7: Generation of 1-amino-compounds at different temperatures and their derivatization with a fluorescent dye. Panel A shows the elution profile of G0F glycans obtained at 37°C. The first isotopic peak for glycosylamine and 1-amino-glycitol overlap indicating the presence of glycosylamine only. Panel B reveals the occurrence of mainly the 1-amino-glycitol upon release at 60°C with 2 M borohydride. The glycosylamine is clearly separated. Panel C shows the elution pattern of the major N-glycans in human IgG. Panel D is a high-resolution spectrum of the major compound with 1 galactose (termed G1 F or AGnF) showing peaks for both the glycosylamine and the reduced form 1-amino-glycitol.

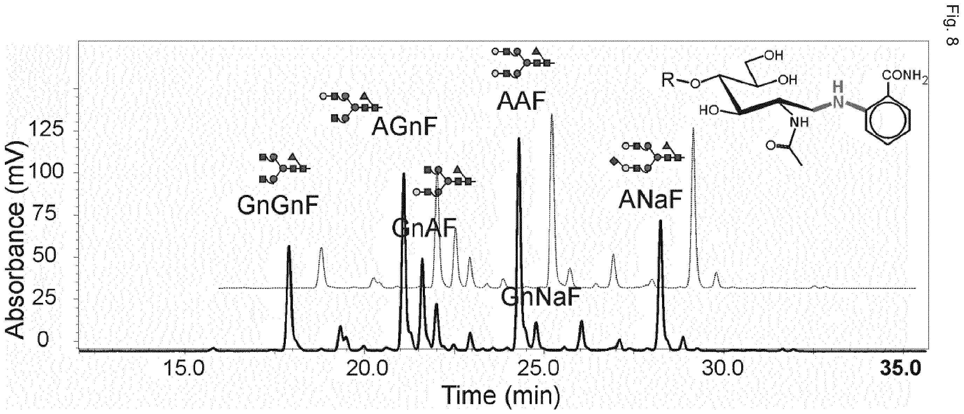

Fig. 8: HPLC analysis of N-glycans from human IgG generated either by reductive alkaline release at 37°C (bold graph) or PNGase F (faint, dotted line). The released glycans were derivatized by reductive amination with 2-aminobenzamide, which reacts with aldehyde groups of reducing sugars. The glycan profiles are essentially identical.

### Description of embodiments

Glycosylation is the most abundant protein post-translational modification, playing an important role in protein folding, stability, and function. Current methods for glycosylation analysis do not allow for a simultaneous assessment of the protein and the glycan portion of a glycosylated protein, which is a major drawback for the investigation of glycosylation on a proteomic scale, as well as for engineering and quality control of therapeutic proteins. Based on the emerging market of protein therapeutics, most of which rely on correct glycosylation for efficacy and safety, and glycoprotein-based diagnostics, there is a significant need today for improved technologies that can routinely be applied in both a clinical and a research setting for the analysis of protein glycosylation.

The release of N-glycans from glycoproteins usually is achieved with (expensive) enzymes or (poisonous and explosive) hydrazine, which produce reducing glycans, or - in the case of enzymes - transiently glycosylamines. For glycoprotein analysis, reducing sugars are reductively aminated. Alternatively, a very new method for derivatizing glycosylamines is employed.

The present invention relates to an improved method of reductive alkaline hydrolysis. This method does not only generate the expected a) reduced glycitols but also substantial amounts of b) glycosylamines, c) reducing N-glycans and d) reduced 1-amino-glycitols.

The lack of a reactive aldehyde group in reduced oligosaccharides prevents their use in many applications such as fluorescent labeling or array printing. They are, however, the perfect format for LC-ESI-MS analysis of N- and O-glycans (Jensen, P.H., Karlsson, N.G., et al. 2012) (Kolarich, D., Windwarder, M., et al. 2015, Pabst, M. and Altmann, F. 2011).

Here a somewhat forgotten mode of N-glycan release is reconditioned. It is shown that all classes of N-glycans, including sialylated and core fucosylated, both 3-or 6-linked, were effectively released. Near complete recovery of oligosaccharides was observed when rather pure glycopeptides were subjected to chemical release with improved parameters of the classical Carlson conditions.

As used herein, the terms "glycoprotein" or "glycopeptide" refer to a protein that contains a peptide backbone covalently linked to one or more glycans (i.e., sugar moieties). Glycoproteins can contain O-linked glycans and/or N-linked glycans. Each glycoprotein molecules comprises a polypeptide having a particular amino acid sequence (which amino acid sequence includes at least one glycosylation site) and at least one glycan covalently attached to the at least one glycosylation site. Individual molecules of a particular glycoprotein within a glycoprotein preparation typically have identical amino acid sequences but may differ in the occupancy of at least one glycosylation site and/or in the identity of the glycans linked to the at least one glycosylation site. That is, a glycoprotein preparation may contain only a single glycoform of a particular glycoprotein, but more typically contains a plurality of glycoforms. Different preparations of the same glycoprotein may differ in the identity of glycoforms present (e.g., a glycoform that is present in one preparation may be absent from another) and/or in the relative amounts of different glycoforms.

The term "glycan", as used herein, refers to a type of carbohydrate, typically an oligosaccharide or polysaccharide. Glycans typically comprise monosaccharide residues linked by O-glycosidic linkages. In the context of protein glycosylation, the term glycan refers to the carbohydrate portion of a glycoprotein.

Glycans are typically divided into five classes: (i) N-linked glycans are glycans attached to a nitrogen of an asparagine or arginine residue side-chain of proteins or peptides; (ii) O-linked glycans are glycans attached to the hydroxy group oxygen of a serine, threonine, tyrosine, hydroxylysine, or hydroxyproline residue side-chain of proteins or peptides, or to oxygens on lipids such as ceramide; (iii) phospho-glycans are glycans attached to the phosphate moiety of a phospho-serine residue of a protein or peptide; (iv) C-linked glycans are glycans attached to a carbon atom of a tryptophan residue side-chain; and (v) GPI anchors are glycans linking proteins to lipids.

Glycans can be monomers or polymers of sugar residues, but typically contain at least three sugars, and can be linear or branched. A glycan may include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2-deoxyribose, phosphomannose, 6-sulfo-*N-*acetylglucosamine, etc.). The term "glycan" includes homo and heteropolymers of sugar residues. The term "glycan" also encompasses a glycan component of a glycoconjugate (e.g., of a glycoprotein, glycolipid, proteoglycan, etc.). The term also encompasses free glycans, including glycans that have been cleaved or otherwise released from a glycoconjugate.

The term "N-glycan," as used herein, refers to a polymer of sugars that has been released from a glycoconjugate but was formerly linked to the glycoconjugate via a nitrogen linkage. Thus, N-linked glycans are glycans that are linked to a glycoconjugate via a nitrogen linkage. A diverse assortment of N-linked glycans exists, but is typically based on the common core pentasaccharide (Man)₃(GlcNAc)(GlcNAc).

The term "O-glycan," as used herein, refers to a polymer of sugars that has been released from a glycoconjugate but was formerly linked to the glycoconjugate via an oxygen linkage. Thus, O-linked glycans are glycans that are linked to a glycoconjugate via an oxygen linkage. O-linked glycans are typically attached to glycoproteins via N-acetyl-D-galactosamine (GalNAc) or via N-acetyl-D-glucosamine (GlcNAc) to the hydroxyl group of L-serine (Ser) or L-threonine (Thr). Some O-linked glycans also have modifications such as acetylation and sulfation. In some instances O-linked glycans are attached to glycoproteins via fucose or mannose to the hydroxyl group of L-serine (Ser) or L-threonine (Thr).

In some embodiments, the term glycan refers to glycans having a molecular weight of less than about 200 kDa, less than about 150 kDa, less than about 100 kDa, less than about 50 kDa, less than about 40 kDa, less than about 30 kDa, less than about 25 kDa, less than about 20 kDa, less than about 15 kDa, less than about 10 kDa, less than about 9 kDa, less than about 8 kDa, less than about 7 kDa, less than about 6 kDa, less than about 5 kDa, less than about 4 kDa, less than about 3 kDa, less than about 2 kDa, less than about 1.5 kDa, less than about 1 kDa, or less than about 500 Da.

Fucosylated oligosaccharides occur throughout nature and many of them play a variety of roles in biology, especially in a number of recognition processes. The oligosaccharides in mammals are of potential medical importance, particularly in inflammation and cancer. Indeed, changes in fucosylation patterns may be used for diagnoses of some diseases and monitoring the success of therapies. In one embodiment of the invention the glycans the OH-groups at C6 and/or C3 are substituted by fucose.

The method of chemical bond disruption is base elimination, also referred to as alkaline elimination (e.g., for N-glycans, O-glycans, phosphorylation, phosphopantetheinylation). In base elimination, the glycoprotein is contacted with a base, for example, an organic or inorganic base, in an amount efficient to disrupt the respective glycan-polypeptide bond. An input of energy, for example, in the form of heat or microwaves, is used in some embodiments, to shorten exposure times required until bond cleavage is achieved. In some embodiments, base elimination is performed at a pH within the range of about 9 to about 12. In some embodiments, when performing base-induced glycan release, such as O-glycan or N-glycan release, a reducing agent (e.g., a strong reducing agent such as sodium borohydride (NaBH₄) is added to prevent the released glycan from degrading. The reducing agent, such as, e.g., NaBH₄, is used in order to reduce the released glycans into the corresponding alditol/aminitol form.

The inventive method utilizes the breakup of covalent glycan-peptide bond in order to release a reduced glycan from a glycoprotein. Thus, the present invention relates to a chemical method for producing N-glycans and/or reduced glycans from a glycopeptide or glycoprotein comprising reacting the glycopeptide or glycoprotein with borohydride in the presence of a base at a temperature of at least 50 °C for a period sufficient to obtain said reduced glycans. The method relies on a break of covalent glycan-peptide bond in order to release a glycan from a glycoprotein.

As described herein, glycoproteins or glycopeptides may be fully deglycosylated without any apparent adverse effects on their glycan or protein backbones.

Depending on the process parameters, different glycan products could be provided. E.g., by varying the temperature different glycan products are obtained.

Close inspection of the obtained oligosaccharides by matrix-assisted laser desorption ionization mass spectrometry as well as liquid chromatography electrospray-mass spectrometry revealed the very surprising facts that besides the expected oligosaccharitol, 10-30 % of the glycans occurred in reducing form even after 15 h incubation and about one third emerged in the form of 1-amino-1-deoxy-glycitol. While this result corroborates the notion that reductive release of N-glycans proceeds via the 1-amino intermediate -just like the enzymatic process - it revealed that its hydrolysis competes with reduction to 1-amino-oligosaccharitol. Substantial quantities of deoxygenated compound (2-amino-3-(glyco-)carbamoyl-propanol and/or 2-(glyco-)4-diamino-butyric acid) were also observed.

In some embodiments the method as described herein is carried out at a temperature of about 60 °C. At this condition, predominantly reduced glycans of general formula I and II were obtained (see fig. 4). Predominantly reduced N-glycan moieties are obtained. The released reduced N-glycans are clearly separated from the N-glycans, e.g., from glycosylamine. At a temperature of about 60 °C substantially no N-glycans moieties were released.

The term "substantially no" as used in the context of glycan moieties refers to an amount which is below 20 %, below 15%, below 10 %, below 7%, or even below 5% of the total amount of the released moieties.

In some embodiments the method as described herein is carried out in the presence of 2 M borohydride. At this condition, predominantly reduced glycans of general formula I and II were obtained (see Fig. 5). However, in contrast to the process employing 1 M borohydride, the amount is shifted to reduced N-glycans. Even at a temperature of about 50 °C substantially no N-glycan moieties are released.

It will be appreciated that the methods described herein may be used in a variety of applications. In general, any application that requires structural characterization and/or isolation of glycans linked to proteins could be envisaged.

The present invention is particularly useful for providing a pool of reduced glycans separated from the source in which the glycans were generated and/or with which the glycans were previously associated.

Glycan arrays have become a powerful tool for the high-throughput elucidation of interactions of different carbohydrate structures with a wide variety of biological targets, including antibodies, proteins, viruses and cells. This technique is especially suitable for glycomics studies, because arrays present carbohydrate ligands in a manner that mimics interactions at cell-cell interfaces.

As used herein, the term "glycan array" refers to a binding assay in array format that is used to identify agents that interact with any of a number of different glycans linked to the array substrate (referred to herein as "glycan probes." In some embodiments, glycan arrays comprise a number of glycan probes obtained by chemical cleavage of a covalent bond of a glycoprotein. In some embodiments, glycan arrays comprise at least 2, at least 5, at least 10, at least 20, at least 30, at least 40, at least 50, at least 60, at least 70, at least 80, at least 90, at least 100, at least 150, at least 350, at least 1000 or at least 1500 glycan probes. In some embodiments, glycan arrays may be customized to present a desired set of glycan probes.

Glycan arrays may be comprised of a substrate and at least four glycans wherein at least one glycan is a reduced glycan. These glycans may be selected from any known glycans, including those described herein. Glycans may be linked to arrays by linkers, in some cases selected from -O(CH₂)₂CH₂NH₂ and -O(CH₂)₃NHCOCH₂(OCH₂CH₂)₆NH₂.

In some embodiments, the present invention provides methods of obtaining an anti-glycan antibody profile in a sample comprising contacting a glycan array with a sample, obtaining glycan array binding results and preparing an anti-glycan profile based on the glycan array binding results. Such methods may further comprise selecting at least one binding assay, contacting the sample with the binding assay(s), obtaining results and updating the anti-glycan antibody profile based on the results. In some cases, binding assays are selected from alternative glycan arrays, enzyme-linked immunosorbent assays (ELISAs), flow cytometry-based assays and surface plasmon resonance (SPR)-based assays and evanescent-field fluorescence excitation-based assays. These binding assays may be used to assess binding to a modified epitope, such as a chemically modified epitope. Such modified epitopes may include modified saccharides. In such cases, modified saccharides may comprise one or more modified chemical groups.

In some embodiments, the present invention provides a method of obtaining a glycan profile for a sample comprising contacting an array with a sample, obtaining array binding results, and preparing a glycan profile based on the array binding results. Such methods may further comprise selecting at least one other binding assay, analyzing the sample with the binding assay(s), obtaining results, and updating the glycan profile based on those results from the other binding assay(s) (e.g. an alternative array, an ELISA, a flow cytometry-based assay and a SPR-based assay.) In some cases, such binding assays may include anti-glycan antibody arrays. Some binding assays may assess binding to a modified epitope, such as a chemically modified epitope (e.g. a saccharide with one or more chemical groups).

Samples being analyzed may be from *in vitro* or *in vivo* sources. *In vivo* sources may include human subjects and non-human animal subjects. Non-human animal subjects may include mice, rats, rabbits, cats, dogs, pigs, cows, sheep, chicken and monkeys. Samples may be blood, plasma, serum, cells, tissues, organs, mucus, cerebrospinal fluid, saliva and urine.

In some embodiments, the present invention provides methods of diagnosing a disease, disorder and/or condition comprising the use of an anti-glycan antibody profile or a glycan profile according to the present invention. Such diseases, disorders and/or conditions may be cancer or cancer-related indications, immune-related indications, viral indications, cardiovascular indications and/or gastrointestinal indications. Methods of diagnosing cancer or cancer-related indications may comprise the use of anti-glycan antibody profiles comprising anti-tumor associated carbohydrate antigen (TACA) antibody profiles.

In some embodiments, the present invention provides a diagnostic kit comprising one or more glycan arrays of the invention and instructions for use. In some cases, such kits may be used to detect one or more anti-glycan antibodies in a sample.

Entities being analyzed by binding assays may include any protein, glycan, glycoprotein, molecule, nucleic acid, antibody, antibody fragment, cell or tissue. Further, other terms that may be used for entities involved in binding assays include probes (e.g. glycan probes,) components, biomarkers, ligands and sensors.

In some binding assays, interactions between entities may be detected through the use of one or more detectable labels. Detectable labels may be directly associated with an entity being examined or in some cases, detectable labels may be associated with a secondary agent (e.g. a secondary or detection antibody) capable of associating with one or more of the entities subject to analysis.

In some embodiments, binding assays comprise the anchoring or tethering of a first entity, or probe, to a surface or substrate followed by exposure of the first entity with a second entity being examined for its ability to bind and/or for its level of affinity for the first entity. With such binding assays, second entities may be directly associated with a detectable label. In other cases, a secondary agent, associated with a detectable label, is introduced subsequently to exposure of the first entity with the second entity.

In some cases, probes used in binding assays may comprise glycan probes. Such probes may comprise glycans associated directly with a surface or substrate or may comprise glycans attached to a surface or substrate with a linker.

Linkers useful for tethering entities or probes to a surface or substrate may comprise 10, 11, 12, 13, 14, 15 or more atoms. In a further embodiment, a linker may comprise a group of atoms, e.g., 10-1,000 atoms. Such atoms or chemical groups of atoms may include, but are not limited to, carbon atoms, amino groups, alkylamino groups, oxygen atoms, sulfur atoms, sulfoxide groups, sulfonyl groups, carbonyl groups and imine groups. In some embodiments, linkers may comprise an amino acid, peptide, polypeptide or protein. In some embodiments, a moiety bound by a linker may include, an atom, a chemical group, a nucleoside, a nucleotide, a nucleobase, a sugar, a nucleic acid, an amino acid, a peptide, a polypeptide, a protein, a protein complex, a payload (e.g., a therapeutic agent) or a marker (including, but not limited to a chemical, fluorescent, radioactive or bio luminescent marker). Linkers can be used in the present invention in a variety of applications, such as to form multimers or conjugates, as well as to administer a payload, as described herein. Examples of chemical groups that can be incorporated into linkers include, but are not limited to, alkyl, alkenyl, alkynyl, amido, amino, ether, thioether, ester, alkylene, heteroalkylene, aryl, or heterocyclyl, each of which can be optionally substituted, as described herein. Examples of linkers include, but are not limited to, unsaturated alkanes, polyethylene glycols (e.g., ethylene or propylene glycol monomeric units, e.g., diethylene glycol, dipropylene glycol, triethylene glycol, tripropylene glycol, tetraethylene glycol, or tetraethylene glycol), and dextran polymers, Other examples include, but are not limited to, cleavable moieties within the linker, such as, for example, a disulfide bond (-S-S-) or an azo bond (-N=N-), which can be cleaved using a reducing agent or photolysis.

Array substrates may include a variety of materials. In some cases, arrays may be printed on epoxide-derivatized slides. Further, arrays may be printed using any technologies available in the art. In some cases, printing is carried out using a microarray device. Such devices may include, but are not limited to microarrays using linear servo motor technology. Microarrays of the invention may utilize spotting pins for application of glycans to array substrates. Such spotting pins may include, but are not limited to silicon microarray spotting pins. Spotting pins may comprise pin tips that are from about 10 µm to about 200 µm in size (e.g. from about 10 to about 50, from about 25 to about 75, from about 50 to about 100 and from about 75 to about 200 µm). Spotting pins may also comprise volumes of from about 0.05 µm to about 1 µm (e.g. from about 0.05 to about 0.2, from about 0.1 to about 0.5, from about 0.25 to about 0.75, from about 0.5 to about 1.0 µm). Spotting pins of the invention may be used to generate glycan spots with diameters of from about 1 µm to about 500 µm (e.g. from about 1 to about 10, from about 5 to about 50, from about 20 to about 70, from about 50 to about 100, from about 75 to about 150, from about 100 to about 300, from about 200 to about 500 µm).

### Examples

The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

### Materials and Methods

### Glycopeptides and reference glycans

A crude glycopeptide preparation from white beans (haricot beans, origin USA) was obtained by pepsin digestion of bean powder and finally, gel filtration over Sephadex G25. Fractions containing maximal concentrations of glycopeptides were pooled. Also pronase glycopeptide derived from fibrin was applied. Tryptic peptides from human IgG were purified by passage over Sephadex G50.

### Chemical release of N-glycans

In the standard approach, 1.5 mg of glycopeptide was dissolved in 1 mL of 50 mM NaOH containing 1 M NaBH₄. This mixture was incubated at 50 °C for 15 h. Either with or without acidification the sample was then purified using a PGC cartridge (250 mg, Supelclean ENVI-Carb SPE (Solid phase extraction), Sigma-Aldrich)equilibrated with a pH 9.0 buffer (65 mM ammonium formate). Glycans were eluted with 50 % acetonitrile (v/v) in the same buffer. Other conditions for these steps were applied as indicated in the results section.

The eluate was dried in a rotary evaporator and taken up in water or alkaline buffer

### Separation of 1-amino-1-deoxy-glycitol

100 mg of peptic fibrin glycopeptides were subjected to the standard chemical release and acidified with dilute acetic acid. The resulting glycans were desalted by gel filtration on a Sephadex G25 medium with 2 % acetic acid as the eluent. Orcinol positive fractions were pooled and applied to a column with Dowex 50 (15 mL; VWR Vienna) equilibrated with 2 M acetic acid. After elution of all unbound material - including reduced and non-reduced glycans-with three column volumes of 2 % acetic acid, the amino-glycans were eluted with 0.1 M ammonium acetate of pH 6.0. Amino-glycitols were desalted and separated from peptidic material by passage over a porous graphitic carbon cartridge as described above.

### Mass spectrometric analysis

For MS analysis, the desalted reaction mixtures were routinely analyzed by matrix assisted-time of flight mass spectrometry (MALDI-TOF MS) with 2,5-dihydroxybenzoic acid as matrix and a Bruker Autoflex MALDI-TOF MS in positive ion reflectron mode.

Some samples were subjected to liquid chromatography-electrospray ionization mass spectrometry (LC-ESI-MS) with a porous graphitic carbon (PGC) column and a Bruker amaZon ion trap as the detector. Solvents and gradient were as described previously (Pabst, M. and Altmann, F. 2011; Pabst, M., Grass, J., et al. 2012).

### RESULTS

### Initial trials

In recent work on O-glycans of human cells (Pabst, M., Wu, S.Q., et al. 2010), it was observed that the reductive alkaline release (RAR) of O-glycans was accompanied by a considerable bycatch of free N-glycans. In order to determine whether RAR - though sunken into oblivion for more than two decades - could be a cost-effective approach for the preparation of reduced glycans (glycitols) that can be used as standards for N-glycan analysis by PGC-LC-MS (Jensen, P.H., Karlsson, N.G., et al. 2012, Pabst, M. and Altmann, F. 2011), trials have been initiated. Glycopeptides from very affordable sources, i.e. white beans or bovine fibrin, were treated with alkaline borohydride with the classical Carlson conditions. In order to allow comparison of different conditions, the reaction mixtures were just passed over PGC cartridges and the eluate was analyzed by MALDI-TOF-MS (Fig. 1).

The first lesson to learn was that sample purity markedly affected the results. Glycopeptide preparations containing much other peptide material gave very bad MALDI spectra with the glycans hardly visible. Rather pure glycopeptide (estimated purity of over 50 %), however, yielded spectra comparable to a PNGase F digest, albeit with some additional peaks (Fig. 1), some of which later being identified as reaction intermediates.

### Interpretation of the isotopic pattern of released glycans

A 16 h treatment with 1 M borohydride at elevated temperature is expected to generate reduced glycans = glycitols. Zooming into the individual peaks of MMXF, however, revealed a totally distorted isotopic profile, which in addition to the expected mass of the glycitol + Na+ ion (m/z = 1213.43) exhibited large peaks lighter by 1 and 2 Da (Fig. 2). Similar unexpected peak profiles were found for all other peaks. The assumption of a reducing end was verified in several ways. First, increasing the borohydride concentration greatly diminished the height of the m/z = 1211.42 peak (Fig. 2B); second, a separate reduction step after purification of the products eliminated this peak and third, PGC-LC gave two peaks as expected for the α and β anomer of a reducing glycan. The second peak (m/z = 1212.45), however, was much larger than attributable to the reduced glycans (Fig. 2A).

This peak in part may represent the monoisotopic mass of 1-amino-1-deoxy-glycit, i.e. the reduced glycosylamine, which is assumed to be the initial product of reductive cleavage of the N-glycosidic bond (Likhosherstov, L.M., Novikova, O.S., et al. 1990). If that were true, this amino-glycit should be separable by cation exchange chromatography.

To this end, a preparative chemical degradation of fibrin glycopeptides (essentially A⁴A⁴ glycan with a GENR peptide moiety and two terminal Galβ1,4-residues) was applied to a Dowex 50 cation exchange resin. Indeed, a substantial amount of the sample bound to the resin. The flow through fraction when analyzed by PGC-LC-ESI-MS contained reduced glycan (glycitol) plus reducing glycans eluting as two anomers. The binding fraction contained the expected glycan of m/z = 822.814, but in addition also a considerable amount of glycans with - apparently - a normal reducing end. As this fraction did not contain any glycitol, insufficient washing could not explain the reducing sugars in the cationic fraction. However, unlike for previous experiments, the PGC clean-up for this preparation had been conducted at pH 9 and the sample was rapidly processed with the cation-exchange resin at acidic pH. Here, however, a possibly present glycosylamine would have degraded to the reducing sugar. In fact, MALDI-TOF MS as well as PGC-LC-ESI-MS at pH 9.0 of the unseparated mixture revealed the presence of a peak 1 Da smaller than the reducing glycan (Fig. 3).

Most analyses contained peaks 100 Da larger than the reducing glycan, e.g. the prominent peak of m/z = 1311.32 in the bean N-glycan or the m/z = 961.35 peak in the fetuin sample. MS/MS spectra confirmed their glycan nature. These compounds can be explained as reaction intermediates still containing the asparagine but with a hydroxyl-group at C1 (2-amino-3-(glyco-)carbamoyl-propanol). That reminds of the chemical mechanism of reductive alkaline cleavage of peptide bonds (Benisek, W.F., Raftery, M.A., et al. 1967), which in turn also leads to reduction of the side chain carboxyl group of glycosylated Asn (Likhosherstov, L.M., Novikova, O.S., et al. 1990).

Fetuin glycans revealed yet another modification, i.e. deacetylation, with sialic acids apparently being particularly prone to this degradation. GlcNAc residues underwent deacetylation only at elevated temperatures.

### Varying the conditions

As the release conditions originally supposed by Carlson (Carlson, D.M. 1968) were optimized for O-glycans, the example of others was followed (Argade, S.P., Daves, G.D., Jr., et al. 1989) and the conditions were varied (Fig. 3). These experiments were done with the A⁴A⁴-GENR glycopeptide, basic conditions for clean-up and basic solvent for PGC-LC-ESI-MS throughout. MALDI-TOF-MS was also carried out bearing in mind that the acidic matrix would cause some decay of glycosylamine. Four temperatures between 37 and 70 °C were employed. MALDI spectra show that at 37 °C some glycopeptide material was still present. This incomplete glycan release was reflected by the lower total peak volume of the released glycan in PGC-LC-ESI-MS, which quantitative estimates. The four products eluted over a narrow time window and thus the sum isotope pattern is shown (Fig. 3).

The dominant peak in the oligosaccharide region had m/z= 820.80 and was thus the glycosylamine. The glycosylamine peak shrunk rapidly with increasing temperature indicating a strong temperature dependence of its hydrolysis at alkaline pH. As the isotope patterns of the four products overlapped, the individual shares were sort of deconvoluted using the theoretical isotope pattern as model. The dependence of product nature on temperature is displayed in Fig. 4. The most distinctive result was the significantly different yield of glycosylamine at different temperatures. Notably, glycosylamine will rapidly decompose to the regular reducing sugar at neutral to acidic pH.

When glycitol is the desired product, release at 60 °C constitutes a good compromise between complete reduction and only marginal de-N-acetylation. Should the stable aminitol be the desired product, higher temperature and higher borohydride concentration are the right choice.

### Estimation of the degree of release

As several references point at a low yield upon alkaline release under Carlson conditions (Argade, S.P., Daves, G.D., Jr., et al. 1989, Hounsell, E.F., Pickering, N.J., et al. 1984, Ogata, S. and Lloyd, K.O. 1982), the peak area of the fibrin glycan obtained by chemical versus enzymatic release were compared with LC-ESI-MS. Surprisingly, the area of the enzyme liberated glycan peaks was smaller, which can be explained by the fact that the pronase glycopeptides in part contained terminal Asn-residues. These glycopeptides were not fully degraded. The fact that no such undegraded glycopeptides remained at temperatures from 50 °C onwards - except from the glyco-asparaginol - also pointed at a quantitative release. Evidence in the same direction was the fact that higher temperatures or prolonged incubation at 60 °C did not cause a decrease in total peak area. Thus, no post-release degradation occurred.

### Separation of the 1-amino-oligosaccharitol (1-amino-glycitol) by cation exchange

A crude glycopeptide preparation was obtained from bovine fibrin (Sigma-Aldrich) by digestion with pronase (Protease from *Streptomyces griseus;* Sigma-Aldrich) and subsequent separation from smaller peptides by gel filtration on Sephadex G25 fine using 1 % acetic acid as the solvent. The carbohydrate positive fraction (orcinol test) was freeze dried.

A 100 mg portion of this fibrin glycopeptide was subjected to chemical deglycosylation at 60 °C (50 mM NaOH, 1 M NaBH₄) for 16 h. The preparation was acidified with 4 M acetic acid and desalted by passage over Sephadex G-25 medium with 1 % acetic acid as the eluent. Glycan containing fractions were pooled and concentrated under reduced pressure. Amino-terminated glycans were separated from other variants by application to a Dowex 50 cation exchange column (15 mL; Biorad, USA) previously equilibrated with 2 % acetic acid. After washing with 20 mL of 2 % acetic acid, bound material was eluted with 0.1 M NH₄Ac of pH 6.0. Fractions of 1 mL were collected and analyzed for carbohydrate. A prominent peak eluting in fractions 26 and 27 was assumed to contain 1-amino-glycitol.

Analysis of the two glycan-containing fractions by LC-ESI-MS using a porous graphitic carbon (PGC) column revealed one peak each. A glycan with m/z = 822.37 (Hex₅HexNAc₄) in the first peak representing the expected reduced oligosaccharide and a glycan with m/z = 821.88 indicative of 1-amino-glycitol.

### Derivatization with an amine-specific reagent

An IgG glycopeptide preparation was obtained by pronase digestion as described above. 1 mg of this glycopeptide was subjected to chemical release under two different conditions.

Trial 1 a: Release at 37 °C with 0.5 M NaBH₄ in 100 mM NaOH to obtain glycosylamines, and
Trial 1b: Release at 60 °C with 2 M NaBH₄ in 50 mM NaOH to obtain 1-amino-glycitol.

In both cases, the samples were incubated for 16 h and then passed over a cartridge filled with porous graphitic carbon (10 mg, Hypercarb, Thermo Scientific) in 50 mM NaHCO₃ (pH 8.4) (to preserve glycosylamines when present). Elution of bound glycans was achieved by the addition of 50 % acetonitrile (v/v) to the bicarbonate solution. The eluate was dried in a rotary evaporator and taken up in 20 µL of bicarbonate solution. 5 µL InstantPC dye (Prozyme, Hayward, CA, USA). After 5 min incubation at room temperature, 200 µL of 1 % formic acid in acetonitrile was added and the sample was applied to a Chromabond CN cartridge (100 mg, Macherey & Nagel, Düren, Germany) previously equilibrated with 1 % formic acid in acetonitrile, which was also used to wash out unbound material. Glycans are eluted with 10 % acetonitrile in water.

Analysis of InstantPC labeled glycans was accomplished with HILIC (hydrophilic interaction) chromatography on an amide-silica capillary column (Inertsil Amide, 5 micron, Capillary-EX Column, 150 x 0.3 mm; GL Sciences, Torrance, CA, USA). Solvent A was 80 mM formic acid buffered to pH 3.0 with ammonia and solvent B was 80 % acetonitrile in solvent A. A gradient from 99 to 65 % B was developed over 45 min at a flow rate of 6 µL/min and a column temperature of 31 °C. InstantPC labeled glycans were analyzed by ESI-MS using an amaZon ion trap or a Maxis 4G Q-TOF instrument (Bruker, Bremen, Germany).

Trial 1a yielded one InstantPC labeled product with the mass of a glycosylamine (m/z = 862.8). The mass indicative of the 1-amino-glycitol (863.8) occurred only in the isotopic tail of the glycosylamine (Fig. 7, panel A).

Trial 1 b in contrast gave the 1-amino-glycitol as the major product with just some remaining glycosylamine. The two products were clearly separated by the HILIC column (Fig. 7, panel B). The major glycoforms of IgG were found as InstantPC labeled 1-amino-glycitols (Fig. 7, panel C). Panel D depicts the high-resolution mass spectrum of compound "G1F" (Gal1GlcNAc₄Man₃Fuc₁) as glycosylamine (calc. m/z = 943.8725) or 1-amino-glycitol (calc. m/z = 945.8882).

### Reductive amination of reducing sugars generated by reductive alkaline release

The release of glycans was done as described for trial 1a, but the subsequent clean-up was performed with acidic buffer (80 mM formic acid brought to pH 3.0 with ammonia). Thereby, glycosylamines decay to reducing sugars. These were derivatized with 2-aminobenzamide by reductive amination (Ahn J. et al., Separation of 2-aminobenzamide labeled glycans using hydrophilic interaction chromatography columns packed with 1.7 µm sorbent; Journal of Chromatography B, 878 (2010) 403-408) and analyzed by HILIC chromatography using an "AdvancedBio Glycan Mapping Column" (2.1 x 150 mm, 1.8 µm; Agilent, Waldbronn, Germany).

A parallel sample was prepared by digestion of the glycopeptides with peptide:N-glycanase F (PNGase F).

A comparison of AB-labeled N-glycans from human IgG generated either by reductive alkaline release (bold graph) or PNGase F (faint, dotted line) is shown in Fig. 8.

### CONCLUSIONS

Opposite to what reasoning and literature suggest (Ogata, S. and Lloyd, K.O. 1982), the major initial products of reductive alkaline release of N-glycans at moderate temperatures are not reduced glycitols but unreduced sugars, either as regular reducing sugars or as glycosylamines. At higher temperatures the pendula swings to the reduced 1-amino-glycit and regular glycits = reduced-glycans. The yield of products can further be influenced by the concentration of alkali, which hinders reduction or borohydride, which furthers reduction.

Amine-terminating compounds are especially interesting as they can be targeted by stable fluorogenic reagents (Lauber, M.A., Yu, Y.Q., et al. 2015) or transient (Kamoda, S., Nakano, M., et al. 2005, Yamada, K., Hirabayashi, J., et al. 2013) that allow HPLC analysis and isolation of individual compounds or - on the other hand - coupling to carriers.

### REFERENCES

Argade, S.P., Daves, G.D., Jr., et al. (1989) The effect of alkaline borohydride treatment on N-linked carbohydrates of glycoproteins. Glycoconj J, 6, 45-56.
Benisek, W.F., Raftery, M.A., et al. (1967) Reductive cleavage of acylproline peptide bonds. Biochemistry, 6, 3780-3790.
Carlson, D.M. (1968) Structures and immunochemical properties of oligosaccharides isolated from pig submaxillary mucins. J Biol Chem, 243, 616-626.
Debray, H., Strecker, G., et al. (1984) Effect of alkalis on N-glycosidic linkages of glycoproteins. Biochem Soc Trans, 12, 611-612.
Hounsell, E.F., Pickering, N.J., et al. (1984) The effect of mild alkali and alkaline borohydride on the carbohydrate and peptide moieties of fetuin. Biochem Soc Trans, 12, 607-610.
Jensen, P.H., Karlsson, N.G., et al. (2012) Structural analysis of N- and O-glycans released from glycoproteins. Nat Protoc, 7, 1299-1310.
Kamoda, S., Nakano, M., et al. (2005) Rapid and sensitive screening of N-glycans as 9-fluorenylmethyl derivatives by high-performance liquid chromatography: a method which can recover free oligosaccharides after analysis. J Proteome Res, 4, 146-152.
Karlsson, N.G. and Packer, N.H. (2002) Analysis of O-linked reducing oligosaccharides released by an in-line flow system. Anal Biochem, 305, 173-185.
Kolarich, D., Windwarder, M., et al. (2015) Isomer-Specific Analysis of Released N-Glycans by LC-ESI MS/MS with Porous Graphitized Carbon. Methods Mol Biol, 1321, 427-435.
Lauber, M.A., Yu, Y.Q., et al. (2015) Rapid Preparation of Released N-Glycans for HILIC Analysis Using a Labeling Reagent that Facilitates Sensitive Fluorescence and ESI-MS Detection. Anal Chem, 87, 5401-5409.
Likhosherstov, L.M., Novikova, O.S., et al. (1990) A selective method for sequential splitting of O- and N-linked glycans from N,O-glycoproteins. Carbohydr Res, 199, 67-76.
Ogata, S. and Lloyd, K.O. (1982) Mild alkaline borohydride treatment of glycoproteins-a method for liberating both N- and O-linked carbohydrate chains. Anal Biochem, 119, 351-359.
Pabst, M. and Altmann, F. (2011) Glycan analysis by modern instrumental methods. Proteomics, 11, 631-643.
Pabst, M., Grass, J., et al. (2012) Isomeric analysis of oligomannosidic N-glycans and their dolichol-linked precursors. Glycobiology, 22, 389-399.
Pabst, M., Wu, S.Q., et al. (2010) IL-1beta and TNF-alpha alter the glycophenotype of primary human chondrocytes in vitro. Carbohydr Res, 345, 1389-1393.
Rasilo, M.L. and Renkonen, O. (1981) Mild alkaline borohydride treatment liberates N-acetylglucosamine linked oligosaccharide chains of glycoproteins. FEBS Lett, 135, 38-42.
Schulz, B.L., Packer, N.H., et al. (2002) Small-scale analysis of O-linked oligosaccharides from glycoproteins and mucins separated by gel electrophoresis. Anal Chem, 74, 6088-6097.
Spiro, R.G. and Bhoyroo, V.D. (1974) Structure of the O-glycosidically linked carbohydrate units of fetuin. J Biol Chem, 249, 5704-5717.
Yamada, K., Hirabayashi, J., et al. (2013) Analysis of O-glycans as 9-fluorenylmethyl derivatives and its application to the studies on glycan array. Anal Chem, 85, 3325-3333.
Yu, G., Zhang, Y., et al. (2010) Effect and limitation of excess ammonium on the release of O-glycans in reducing forms from glycoproteins under mild alkaline conditions for glycomic and functional analysis. Anal Chem, 82, 9534-9542.

## Claims

1. A method for producing reduced glycans from a glycopeptide or glycoprotein comprising reacting the glycopeptide or glycoprotein with borohydride in the presence of a base at a temperature of at least 50 °C for a period sufficient to obtain the reduced glycans.

2. The method according to claim 1, wherein the reduced glycans are of general formula I and/or II 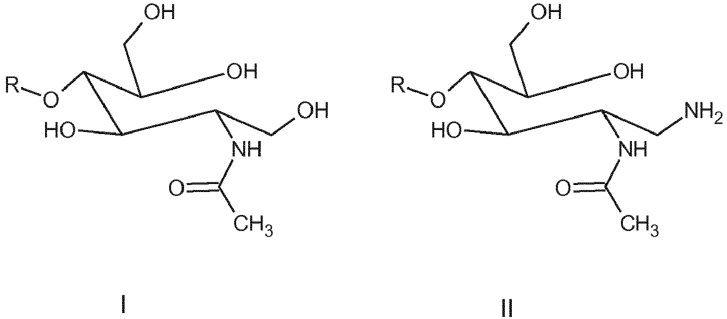 wherein R denotes a glycan residue, and optionally the OH-groups at C6 and/or C3 may be substituted by fucose.

3. The method according to claim 1 or 2, wherein the temperature is about 55 to 90 °C, or about 60 to 80 °C.

4. The method according to any one of claims 1 to 3, wherein the borohydride is used in a concentration of about 0.5 to 3.0 M, or of about 0.8 to 2.0 M.

5. The method according to any one of claims 1 to 4, wherein the base is used in a concentration of about 0.01 to 3.0 M, or of about 0.05 to 2.0 M.

6. The method according any one of claims 1 to 5, wherein the base is NaOH, KOH, or LiOH.

7. The method according to claim 1, wherein the reduced glycans and glycosylamine are obtained.

8. The method according to claim 1, wherein a deoxygenated glyco-asparagine (2-amino-3-(glyco-)carbamoyl-propanol and/or 2-(glyco-)4-diamino butyric acid) is obtained.

9. A compound of general formula II, III or IV, 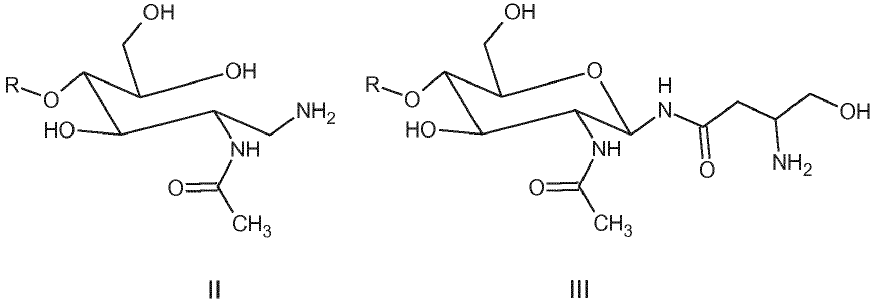 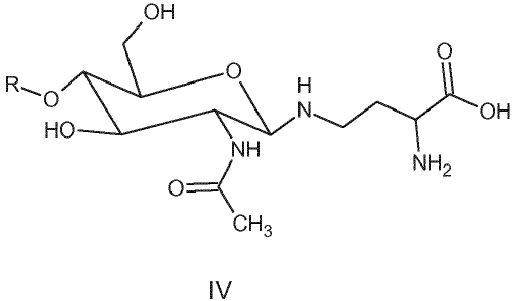 wherein R is a glycan residue, wherein optionally the OH-groups at C6 and/or C3 may be substituted by fucose.

10. A glycan array comprising:
a substrate, and
at least four glycans, wherein at least one glycan is a compound of general formula II or III.

11. The glycan array according to claim 10, wherein each glycan is attached to said substrate by a linker.

12. The glycan array according to claim 11, wherein said linker is selected from the group consisting of -O(CH₂)₂CH₂NH₂ and -O(CH₂)₃NHCOCH₂(OCH₂CH₂)₆NH₂.

13. Use of a glycan array according to any of claims 10 to 12 in biochemical research or for biomedical applications.

14. Use according to claim 13, wherein the binding of proteins to glycans of different structure is characterized.

15. A stationary phase for affinity chromatography, wherein a compound of general formula II or III is bound to the chromatography matrix.
